# EUROPEAN PATENT APPLICATION

(11) **EP 1 882 475 A1**
(43) Date of publication of application: **30.01.2008**
(21) Application number: 06117874.5
(22) Date of filing: 26.07.2006
(51) Int. Cl.: A61K 31/505, A61K 31/506, A61K 31/53, A61P 19/00, A61P 19/02, A61P 19/04, A61P 19/08, A61P 27/02, A61P 35/00, A61P 17/00, A61P 11/00, A61P 3/04, A61P 3/10

(54) **Method of treating disorders mediated by the fibroblast growth factor receptor**

(71) Applicant: Novartis AG, 4056 Basel (CH)
(72) Inventor: The designation of the inventor has not yet been filed
(74) Representative: Roth, Peter Richard

(57) **Abstract**

The invention relates to a method of treating a warm-blooded animal having a disorder mediated by the fibroblast growth factor receptor (FGFR), in particular 8p11 myelo-proliferative syndrome (EMS), pituitary tumors, retinoblastoma, synovial sarcoma, chronic obstructive pulmonary disease (COPD), seborrheic keratosis, obesity, diabetes and related disorders, autosomal dominant hypophosphatemic Rickets (ADHR), X-chromosome linked hypophosphatemic rickets (XLH), tumor-induced osteomalacia (TIO) and fibrous dysplasia of the bone (FD) as well as to a method of promoting localized neochondrogenesis comprising administering to the warm-blooded animal a urea derivatives of formula (I) 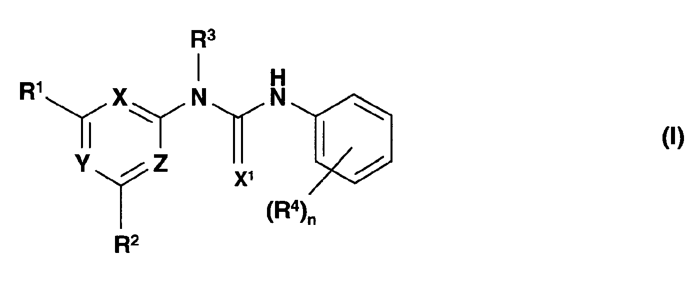 wherein the radicals and symbols have the meanings as defined herein.

## Description

The invention relates to a method of treating a warm-blooded animal having a disorder mediated by the fibroblast growth factor receptor (FGFR), in particular 8p11 myeloproliferative syndrome (EMS), pituitary tumors, retinoblastoma, synovial sarcoma, chronic obstructive pulmonary disease (COPD), seborrheic keratosis, obesity, diabetes and related disorders, autosomal dominant hypophosphatemic Rickets (ADHR), X-chromosome linked hypophosphatemic rickets (XLH), tumor-induced osteomalacia (TIO) and fibrous dysplasia of the bone (FD) as well as to a method of promoting localized neochondrogenesis.

Normal growth, as well as tissue repair and remodeling, require specific and delicate control of activating growth factors and their receptors. Fibroblast Growth Factors (FGFs) constitute a family of over twenty structurally related polypeptides that are developmentally regulated and expressed in a wide variety of tissues. FGFs stimulate proliferation, cell migration and differentiation and play a major role in skeletal and limb development, wound healing, tissue repair, hematopoiesis, angiogenesis, and tumorigenesis (reviewed in Ornitz, Novartis Found Svmp 232: 63-76; discussion 76-80, 272-82 (2001)).

W02006/000420 discloses urea derivatives of formula (I) as further defined below being inhibitors of the FGFR kinase activity.

It was now found that urea derivatives of formula (I) as set forth and defined in W02006/000420 are suitable to treat the disorders mentioned herein. Hence, the present invention relates to a method of treating a warm-blooded animal having a disorder mediated by the fibroblast growth factor receptor (FGFR), in particular 8p11 myeloproliferative syndrome (EMS), pituitary tumors, retinoblastoma, synovial sarcoma, chronic obstructive pulmonary disease (COPD), seborrheic keratosis, obesity, diabetes and related disorders, autosomal dominant hypophosphatemic Rickets (ADHR), X-chromosome linked hypophosphatemic rickets (XLH), tumor-induced osteomalacia (TIO) or fibrous dysplasia of the bone (FD) comprising administering to the warm-blooded animal a urea derivatives of formula (I) 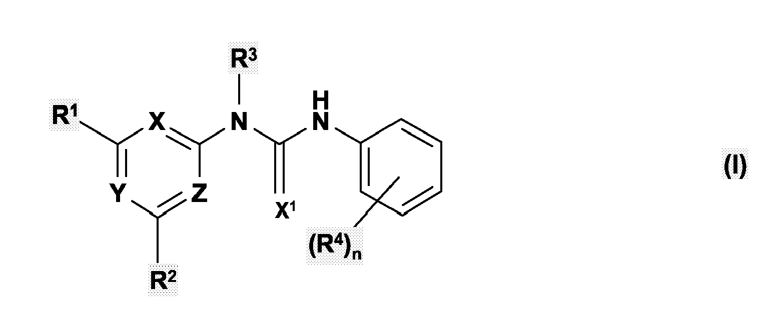 wherein
n is 0, 1, 2, 3, 4 or 5;
X, Y and Z are each independently selected from N or C-R⁵, wherein at least two of X, Y and Z are N; and
X¹ is oxygen,
R¹, R², R³ and R⁴ if present, are each independently selected from an organic or inorganic moiety,
where the inorganic moiety is especially selected from halo, especially chloro, hydroxyl, cyano, azo (N=N=N), nitro; and
where the organic moiety is substituted or unsubstituted and may be attached via a linker, -L¹-, the organic moiety being especially selected from hydrogen; lower aliphatic (especially C₁, C₂, C₃ or C₄ aliphatic) e.g. lower alkyl, lower alkenyl, lower alkynyl; amino; guanidino; hydroxyguanidino; formamidino; isothioureido; ureido; mercapto; carboxy; sulfo; sulfamoyl; carbamoyl; C(O)H or other acyl; acyloxy; substituted hydroxy; a substituted or unsubstituted cyclic group, for example the cyclic group (whether substituted or unsubstituted) may be cycloalkyl, e.g. cyclohexyl, phenyl, pyrrole, imidazole, pyrazole, isoxazole, oxazole, thiazole, pyridazine, pyrimidine, pyrazine, pyridyl, indole, isoindole, indazole, purine, indolizidine, quinoline, isoquinoline, quinazoline, pteridine, quinolizidine, piperidyl, piperazinyl, pyrollidine, morpholinyl or thiomorpholinyl and, for example, substituted lower aliphatic or substituted hydroxy may be substituted by such substituted or unsubstituted cyclic groups,
and -L¹- has 1, 2, 3, 4 or 5 in-chain atoms (e.g. selected from C, N, O and S) and optionally being selected from (i) C₁, C₂, C₃ or C₄ alkyl, such an alkyl group optionally being interrupted and/or terminated by an -O-, -C(O)- or -NR^{a}- linkage; -O-; -S-; -C(O)-; cyclopropyl (regarded as having two in-chain atoms) and chemically appropriate combinations thereof; and -NR^{a}-, wherein R^{a} is hydrogen, hydroxy, hydrocarbyloxy or hydrocarbyl, wherein hydrocarbyl is optionally interrupted by an -O- or -NH- linkage and may be, for example, selected from an aliphatic group (e.g. having 1 to 7 carbon atoms, for example 1, 2, 3, or 4), cycloalkyl, especially cyclohexyl, cycloalkenyl, especially cyclohexenyl, or another carbocyclic group, for example phenyl; where the hydrocarbyl moiety is substituted or
unsubstituted;
wherein R¹ can also represent -X5NR₇R₈, -X₅NR₇X₅NR₇R⁸, -X5NR₇X5C(O)OR₈, - X5OR₇, -X5R₇ and -X5S(O)₀₋₂R₇; wherein X5 is a bond or C₁₋₄alkylene optionally substituted by 1 to 2 C₁₋₆alkyl radicals; R₇ is selected from hydrogen, C₁₋₆alkyl, C₆₋₁₀aryl-C₀₋₄alkyl, C₅₋₁₀heteroaryl-C₀₋₄alkyl C₃₋₁₀cycloalkyl-C₀₋₄alkyl and C₃₋₁₀heterocycloalkyl-C₀₋₄alkyl ; and R₈ is selected from hydrogen and C₁₋₆alkyl; or R₇ and R₈ together with the nitrogen to which R₇ and R₈ are both attached form heteroaryl or heterocycloalkyl;
wherein any aryl, heteroaryl, cycloalkyl and heterocycloalkyl of R₇ or the combination of R₇ and R₈ can be optionally substituted with 1 to 3 radicals independently selected from halo, nitro, cyano, hydroxy, C₁₋₆alkyl, C₁₋₆alkoxy, halo-substituted-alkyl, halo-substituted-alkoxy, - X5NR₉R₁₀, -X5OR₉, -X5NR₉S(O)₂R₁₀, -X5NR₉S(O)R₁₀, -X5NR₉SR₁₀, -X5C(O)NR₉R₁₀, - X5NR₉C(O)NR₉R₁₀, -X5NR₉C(O)R₁₀, -X5NR₉X5NR₉R₁₀, -X5NR₉X5OR₉, - X5NR₉C(=NR₉)NR₉R₁₀, -X5S(O)₀₋₂R₁₁, -X5NR₉C(O)R₁₀, -X5NR₉C(O)R₁₁, -X5R₁₁, - X5C(O)OR₁₀, -X5S(O)₂NR₉R₁₀, -X5S(O)NR₉R₁₀ and -X5SNR₉R₁₀; wherein X5 is a bond or C₁₋₄alkylene; R₉ and R₁₀ are independently selected from hydrogen and C₁₋₄alkyl; and R₁₁ is C₃₋₁₀heterocycloalkyl optionally substituted with 1 to 3 radicals selected from C₁₋₄alkyl, - X5NR₉X5NR₉R₉, X5NR₉X5OR₉ and -X5OR₉
wherein R₃ can alternatively also represent hydrogen, C₁₋₄alkyl, C₆₋₁₀aryl-C₀₋₄alkyl, C₅₋₁₀heteroaryl-C₀₋₄alkyl C₃₋₁₀cycloalkyl-C₀₋₄alkyl and C₃₋₁₀heterocycloalkyl-C₀₋₄alkyl; wherein any alkyl, aryl, heteroaryl, cycloalkyl or heterocycloalkyl of R₃ is optionally substituted by 1-3 radicals selected from halo, C₁₋₄alkyl, -X5S(O)₀₋₂NR₉R₁₀ and -X5OR₉; wherein X5, R₉ and R₁₀ are as described above;
each R⁴ is the same or different and selected from an organic or inorganic moiety, for example, each R⁴ is the same or different and selected from halogen; hydroxy; protected hydroxy for example trialkylsilylhydroxy; amino; amidino; guanidino; hydroxyguanidino; formamidino; isothioureido; ureido; mercapto; C(O)H or other acyl; acyloxy; carboxy; sulfo; sulfamoyl; carbamoyl; cyano; azo; nitro; C₁-C₇ aliphatic optionally substituted by one or more halogens and/or one or two functional groups selected from hydroxy, protected hydroxy for example trialkylsilylhydroxy, amino, amidino, guanidino, hydroxyguanidino, formamidino, isothioureido, ureido, mercapto, C(O)H or other acyl, acyloxy, carboxy, sulfo, sulfamoyl, carbamoyl, cyano, azo, or nitro; all of the aforesaid hydroxy, amino, amidino, guanidino, hydroxyguanidino, formamidino, isothioureido, ureido, mercapto, carboxy, sulfo, sulfamoyl and carbamoyl groups in turn optionally being substituted on at least one heteroatom by one or, where possible, more C₁-C₇ aliphatic groups, wherein one of the radicals R⁴ can also represent 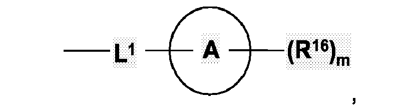 wherein L¹ is a linker; m is 0, 1, 2, 3, 4 or 5; L¹ is a linker; and R¹⁶, if present, are each independently selected from an organic or inorganic moiety,
where the inorganic moiety is especially selected from halo, especially chloro, hydroxyl, cyano, azo (N=N=N), nitro; and
where the organic moiety is substituted or unsubstituted and may be attached via a linker, -L²-, the organic moiety being especially selected from hydrogen; lower aliphatic (especially C₁, C₂, C₃ or C₄ aliphatic) e.g. lower alkyl, lower alkenyl, lower alkynyl; amino; guanidino; hydroxyguanidino; formamidino; isothioureido; ureido; mercapto; C(O)H or other acyl; acyloxy; substituted hydroxy; carboxy; sulfo; sulfamoyl; carbamoyl; a substituted or unsubstituted cyclic group, for example the cyclic group (whether substituted or unsubstituted) may be cycloalkyl, e.g. cyclohexyl, phenyl, pyrrole, imidazole, pyrazole, isoxazole, oxazole, thiazole, pyridazine, pyrimidine, pyrazine, pyridyl, indole, isoindole, indazole, purine, indolizidine, quinoline, isoquinoline, quinazoline, pteridine, quinolizidine, piperidyl, piperazinyl, pyrollidine, morpholinyl or thiomorpholinyl and, for example, substituted lower aliphatic or substituted hydroxy may be substituted by such substituted or unsubstituted cyclic groups,
L¹ and L² each independently being selected from moieties having 1, 2, 3, 4 or 5 in-chain atoms (e.g. selected from C, N, O and S) and optionally being selected from (i) C₁, C₂, C₃ or C₄ alkyl, such an alkyl group optionally being interrupted and/or terminated by an -O-, -C(O)- or -NR^{a}- linkage; -O-; -S-; -C(O)-; cyclopropyl (regarded as having two in-chain atoms) and chemically appropriate combinations thereof; and -NR^{a}-, wherein R^{a} is hydrogen, hydroxy, hydrocarbyloxy or hydrocarbyl, wherein hydrocarbyl is optionally interrupted by an -O- or -NH- linkage and may be, for example, selected from an aliphatic group (e.g. having 1 to 7 carbon atoms, for example 1, 2, 3, or 4), cycloalkyl, especially cyclohexyl, cycloalkenyl, especially cyclohexenyl, or another carbocyclic group, for example phenyl; where the hydrocarbyl moiety is substituted or unsubstituted; or
R₄ is a radical -NHC(O)R₄₅ wherein R₄₅ is selected from C₆₋₁₀aryl-C₀₋₄alkyl, C₅₋₁₀heteroaryl-C₀₋₄alkyl, C₃₋₁₀cycloalkyl-C₀₋₄alkyl and C₃₋₁₀heterocycloalkyl-C₀₋₄alkyl; wherein any aryl, heteroaryl, cycloalkyl or heterocycloalkyl of R₄₅ is optionally substituted with 1 to 3 radicals selected from halo, hydroxy, C₁₋₄alkyl, C₁₋₄alkoxy, halo-substituted-C₁₋₄alkyl, halo-substituted-C₁₋₄alkoxy and C₃₋₈heterocycloC₀₋₄alkyl; wherein any heterocycloalkyl substituent of R₄ is optionally substituted by 1 to 3 C₁₋₄alkyl radicals;
or pharmaceutically acceptable salts, hydrates, solvates, esters, N-oxides protected derivatives, individual isomers and mixture of isomers thereof or prodrugs thereof, in a quantity which is therapeutically effective against said disorder.

The terms employed in the present specification in the definition of the symbols and radicals have the meanings as provided in W02006/000420.

Chromosomal translocations involving the FGF-R1 locus and resulting in activated forms of FGR-R1 have been reported to be responsible for 8p11 myeloproliferative syndrome = Eosinophilic Myeloproliferative Syndrome (EMS) (see D. Macdonald et al., Cross NCP (2002) Acta Haematologica 107: 101-107).

Acidic Fibroblast Growth Factor (especially FGF-1) and FGFR1 have also been described to be involved in aberrant signaling in retinoblastoma, leading to proliferation upon binding of FGF-1 (see e.g. S. Siffroi-Fernandez et al., Arch. Ophthalmology 123, 368-376 (2005)).

The growth of synovial sarcomas has been shown to be inhibited by disruption of the Fibroblast Growth Factor Signaling Pathway (see e.g. T. Ishibe et al., Clin. Cancer Res. 11(7), 2702-2712 (2005)).

Enhanced (especially bronchial) expression of FGFRs, especially FGFR1, has been reported to be associated with Chronic Obstructive Pulmonary Disease (COPD) (see e.g. A. Kranenburg et al., J. Pathol. 206, 28-38 (2005)).

Methods of antagonizing FGFRs, especially FGFR1 or FGFR4, have also been described to be useful in the treatment of obesity, diabetes and/or disorders related thereto, such as metabolic syndrome, cardiovascular diseases, hypertension, aberrant cholesterol and triglyceride levels, dermatological disorders(e.g. infections, varicose veins, Acanthosis nigricans, eczema, exercise intolerance, diabetes type 2, insulin resistance, hypercholesterolemia, cholelithiasis, orthopedic injury, thromboembolic disease, coronary or vascular restriction (e.g. atherosclerosis), daytime sleepiness, sleep apnoea, end stage renal disease, gallbladder disease, gout, heat disorders, impaired immune response, impaired respiratory function, infections following wounds, infertility, liver disease, lower back pain, obstetric and gynecological complications, pancreatitis, stroke, surgical complications, urinary stress incontinence and/or gastrointestinal disorders (see e.g. WO 2005/037235 A2).

Among the diseases promoted by FGFR3 and also other FGFRs (especially in connection with e.g. aberrant FGF23 serum levels), further Autosomal Dominant Hypophosphatemic Rickets (ADHR), X-chromosome linked hypophosphatemic rickets (XLH), tumor-induced Osteomalacia (TIO), fibrous dysplasia of the bone (FD) are to be mentioned (see also X. Yu et al., Cytokine & Growth Factor Reviews 16, 221-232 (2005), and X. Yu et al., Therapeutic Apheresis and Dialysis 9(4), 308-312 (2005)).

A method of promoting localized neochondrogenesis in a cartilage in a mammal comprising administering locally to the cartilage certain kinase inhibitors is described in W02006/038112. Surprisingly, it was found that the compounds of formula (I) as defined herein can be employed in the same manner. Hence, the present invention also relates to a method of promoting localized neochondrogenesis in a cartilage in a mammal comprising administering locally to the cartilage a urea derivatives of formula (I) as defined above or pharmaceutically acceptable salts, hydrates, solvates, esters, N-oxides protected derivatives, individual isomers and mixture of isomers thereof or prodrugs thereof, in a quantity which is effective to promoting localized neochondrogenesis.

It can be shown by established test models that the compounds of formula I are suitable for the treatment of the disorders mentioned herein. The person skilled in the pertinent art is fully enabled to select a relevant test model or study design to prove the hereinbefore and hereinafter indicated therapeutic indications and beneficial effects. Suitable test models and study designs are, for instance, mentioned in the publications cited herein, the disclosure of which is incorporated into the present specification by reference.

The term "method of treatment" or "method of treating" as used herein relates also to a method of prevention of the disorders mentioned herein, i.e. the prophylactic administration of a pharmaceutical composition comprising a compound of formula I to healthy patients to prevent the development of at least one of the disorders mentioned herein.

Suitable pharmaceutical compositions for the treatment of the disorders mentioned herein are disclosed in W02006/000420.

The dosage range of the compound of formula I to be employed depends upon factors known to the person skilled in the art including species of the warm-blooded animal, body weight and age, the mode of administration, the particular substance to be employed and the disease to be treated. Unless stated otherwise herein, the compound of formula I are preferably administered from one to two times per day in a dosage in the range of about 10 to 1000 mg/day.

The present invention also pertains to the use of compound of formula I for the manufacture of a medicament for treating one of the disorders mentioned herein.

## Claims

1. A method of treating a warm-blooded animal having a disorder mediated by the fibroblast growth factor receptor (FGFR) selected from 8p11 myeloproliferative syndrome (EMS), pituitary tumors, retinoblastoma, synovial sarcoma, chronic obstructive pulmonary disease (COPD), seborrheic keratosis, obesity, diabetes and related disorders, autosomal dominant hypophosphatemic Rickets (ADHR), X-chromosome linked hypophosphatemic rickets (XLH), tumor-induced osteomalacia (TIO) and fibrous dysplasia of the bone (FD) comprising administering to the warm-blooded animal a urea derivatives of formula (I) 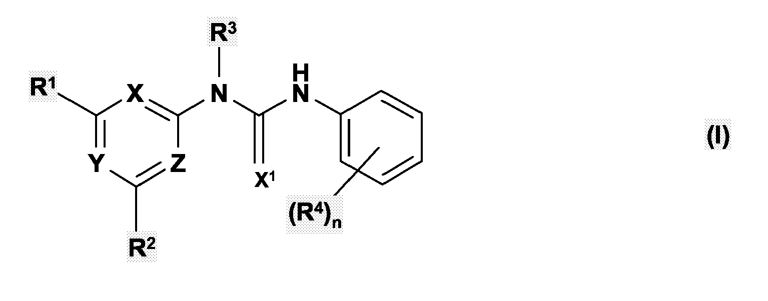 wherein
n is 0, 1, 2, 3, 4 or 5;
X, Y and Z are each independently selected from N or C-R⁵, wherein at least two of X, Y and Z are N; and
X¹ is oxygen,
R¹, R², R³ and R⁴ if present, are each independently selected from an organic or inorganic moiety,
where the inorganic moiety is especially selected from halo, especially chloro, hydroxyl, cyano, azo (N=N=N), nitro; and
where the organic moiety is substituted or unsubstituted and may be attached via a linker, -L¹-, the organic moiety being especially selected from hydrogen; lower aliphatic (especially C₁, C₂, C₃ or C₄ aliphatic) e.g. lower alkyl, lower alkenyl, lower alkynyl; amino; guanidino; hydroxyguanidino; formamidino; isothioureido; ureido; mercapto; carboxy; sulfo; sulfamoyl; carbamoyl; C(O)H or other acyl; acyloxy; substituted hydroxy; a substituted or unsubstituted cyclic group, for example the cyclic group (whether substituted or unsubstituted) may be cycloalkyl, e.g. cyclohexyl, phenyl, pyrrole, imidazole, pyrazole, isoxazole, oxazole, thiazole, pyridazine, pyrimidine, pyrazine, pyridyl, indole, isoindole, indazole, purine, indolizidine, quinoline, isoquinoline, quinazoline, pteridine, quinolizidine, piperidyl, piperazinyl, pyrollidine, morpholinyl or thiomorpholinyl and, for example, substituted lower aliphatic or substituted hydroxy may be substituted by such substituted or unsubstituted cyclic groups,
and -L¹- has 1, 2, 3, 4 or 5 in-chain atoms (e.g. selected from C, N, O and S) and optionally being selected from (i) C₁, C₂, C₃ or C₄ alkyl, such an alkyl group optionally being interrupted and/or terminated by an -O-, -C(O)- or -NR^{a}- linkage; -O-; -S-; -C(O)-; cyclopropyl (regarded as having two in-chain atoms) and chemically appropriate combinations thereof; and -NR^{a}-, wherein R^{a} is hydrogen, hydroxy, hydrocarbyloxy or hydrocarbyl, wherein hydrocarbyl is optionally interrupted by an -O- or -NH- linkage and may be, for example, selected from an aliphatic group (e.g. having 1 to 7 carbon atoms, for example 1, 2, 3, or 4), cycloalkyl, especially cyclohexyl, cycloalkenyl, especially cyclohexenyl, or another carbocyclic group, for example phenyl; where the hydrocarbyl moiety is substituted or unsubstituted;
wherein R¹ can also represent -X5NR₇R₈, -X5NR₇X5NR₇R₈, -X5NR₇X5C(O)OR₈, - X5OR₇, -X5R₇ and -X5S(O)₀₋₂R₇; wherein X5 is a bond or C₁₋₄alkylene optionally substituted by 1 to 2 C₁₋₆alkyl radicals; R₇ is selected from hydrogen, C₁₋₆alkyl, C₆₋₁₀aryl-C₀₋₄alkyl, C₅₋₁₀heteroaryl-C₀₋₄alkyl C₃₋₁₀cycloalkyl-C₀₋₄alkyl and C₃₋₁₀heterocydoalkyl-C₀₋₄alkyl ; and R₈ is selected from hydrogen and C₁₋₆alkyl; or R₇ and R₈ together with the nitrogen to which R₇ and R₈ are both attached form heteroaryl or heterocycloalkyl;
wherein any aryl, heteroaryl, cycloalkyl and heterocycloalkyl of R₇ or the combination of R₇ and R₈ can be optionally substituted with 1 to 3 radicals independently selected from halo, nitro, cyano, hydroxy, C₁₋₆alkyl, C₁₋₆alkoxy, halo-substituted-alkyl, halo-substituted-alkoxy, - X5NR₉R₁₀, -X5OR₉, -X5NR₉S(O)₂R₁₀, -X5NR₉S(O)R₁₀, -X5NR₉SR₁₀, -X5C(O)NR₉R₁₀, - X5NR₉C(O)NR₉R₁₀, -X5NR₉C(O)R₁₀, -X5NR₉X5NR₉R₁₀, -X5NR₉X5OR₉, - X5NR₉C(=NR₉)NR₉R₁₀, -X5S(O)₀₋₂R₁₁, -X5NR₉C(O)R₁₀, -X5NR₉C(O)R₁₁, -X5R₁₁, - X5C(O)OR₁₀, -X5S(O)₂NR₉R₁₀, -X5S(O)NR₉R₁₀ and -X5SNR₉R₁₀; wherein X5 is a bond or C₁₋₄alkylene; R₉ and R₁₀ are independently selected from hydrogen and C₁₋₄alkyl; and R₁₁ is C₃₋₁₀heterocydoalkyl optionally substituted with 1 to 3 radicals selected from C₁₋₄alkyl, - X5NR₉X5NR₉R₉, X5NR₉X5OR₉ and -X5OR₉;
wherein R₃ can alternatively also represent hydrogen, C₁₋₄alkyl, C₆₋₁₀aryl-C₀₋₄alkyl, C₅₋₁₀heteroaryl-C₀₋₄alkyl, C₃₋₁₀cycloalkyl-C₀₋₄alkyl and C₃₋₁₀heterocycloalkyl-C₀₋₄alkyl; wherein any alkyl, aryl, heteroaryl, cycloalkyl or heterocycloalkyl of R₃ is optionally substituted by 1-3 radicals selected from halo, C₁₋₄alkyl, -X5S(O)₀₋₂NR₉R₁₀ and -X5OR₉; wherein X5, R₉ and R₁₀ are as described above;
each R⁴ is the same or different and selected from an organic or inorganic moiety, for example, each R⁴ is the same or different and selected from halogen; hydroxy; protected hydroxy for example trialkylsilylhydroxy; amino; amidino; guanidino; hydroxyguanidino; formamidino; isothioureido; ureido; mercapto; C(O)H or other acyl; acyloxy; carboxy; sulfo; sulfamoyl; carbamoyl; cyano; azo; nitro; C₁-C₇ aliphatic optionally substituted by one or more halogens and/or one or two functional groups selected from hydroxy, protected hydroxy for example trialkylsilylhydroxy, amino, amidino, guanidino, hydroxyguanidino, formamidino, isothioureido, ureido, mercapto, C(O)H or other acyl, acyloxy, carboxy, sulfo, sulfamoyl, carbamoyl, cyano, azo, or nitro; all of the aforesaid hydroxy, amino, amidino, guanidino, hydroxyguanidino, formamidino, isothioureido, ureido, mercapto, carboxy, sulfo, sulfamoyl and carbamoyl groups in turn optionally being substituted on at least one heteroatom by one or, where possible, more C₁-C₇ aliphatic groups, wherein one of the radicals R⁴ can also represent 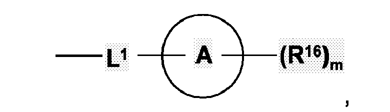 wherein L¹ is a linker; m is 0, 1, 2, 3, 4 or 5; L¹ is a linker; and R¹⁶, if present, are each independently selected from an organic or inorganic moiety,
where the inorganic moiety is especially selected from halo, especially chloro, hydroxyl, cyano, azo (N=N=N), nitro; and
where the organic moiety is substituted or unsubstituted and may be attached via a linker, -L²-, the organic moiety being especially selected from hydrogen; lower aliphatic (especially C₁, C₂, C₃ or C₄ aliphatic) e.g. lower alkyl, lower alkenyl, lower alkynyl; amino; guanidino; hydroxyguanidino; formamidino; isothioureido; ureido; mercapto; C(O)H or other acyl; acyloxy; substituted hydroxy; carboxy; sulfo; sulfamoyl; carbamoyl; a substituted or unsubstituted cyclic group, for example the cyclic group (whether substituted or unsubstituted) may be cycloalkyl, e.g. cyclohexyl, phenyl, pyrrole, imidazole, pyrazole, isoxazole, oxazole, thiazole, pyridazine, pyrimidine, pyrazine, pyridyl, indole, isoindole, indazole, purine, indolizidine, quinoline, isoquinoline, quinazoline, pteridine, quinolizidine, piperidyl, piperazinyl, pyrollidine, morpholinyl or thiomorpholinyl and, for example, substituted lower aliphatic or substituted hydroxy may be substituted by such substituted or unsubstituted cyclic groups,
L¹ and L² each independently being selected from moieties having 1, 2, 3, 4 or 5 in-chain atoms (e.g. selected from C, N, O and S) and optionally being selected from (i) C₁, C₂, C₃ or C₄ alkyl, such an alkyl group optionally being interrupted and/or terminated by an -O-, -C(O)- or -NR^{a}- linkage; -O-; -S-; -C(O)-; cyclopropyl (regarded as having two in-chain atoms) and chemically appropriate combinations thereof; and -NR^{a}-, wherein R^{a} is hydrogen, hydroxy, hydrocarbyloxy or hydrocarbyl, wherein hydrocarbyl is optionally interrupted by an -O- or -NH- linkage and may be, for example, selected from an aliphatic group (e.g. having 1 to 7 carbon atoms, for example 1, 2, 3, or 4), cycloalkyl, especially cyclohexyl, cycloalkenyl, especially cyclohexenyl, or another carbocyclic group, for example phenyl; where the hydrocarbyl moiety is substituted or unsubstituted; or
R₄ is a radical -NHC(O)R₄₅ wherein R₄₅ is selected from C₆₋₁₀aryl-C₀₋₄alkyl, C₅₋₁₀heteroaryl-C₀₋₄alkyl C₃₋₁₀cycloalkyl-C₀₋₄alkyl and C₃₋₁₀heterocycloalkyl-C₀₋₄alkyl; wherein any aryl, heteroaryl, cycloalkyl or heterocycloalkyl of R₄₅ is optionally substituted with 1 to 3 radicals selected from halo, hydroxy, C₁₋₄alkyl, C₁₋₄alkoxy, halo-substituted-C₁₋₄alkyl, halo-substituted-C₁₋₄alkoxy and C₃₋₈heterocycloC₀₋₄alkyl; wherein any heterocycloalkyl substituent of R₄ is optionally substituted by 1 to 3 C₁₋₄alkyl radicals;
or pharmaceutically acceptable salts, hydrates, solvates, esters, N-oxides protected derivatives, individual isomers and mixture of isomers thereof or prodrugs thereof, in a quantity which is therapeutically effective against said disorder.

2. A method of promoting localized neochondrogenesis in a cartilage in a mammal comprising administering locally to the cartilage a urea derivatives of formula (I) as defined in claim 1 or pharmaceutically acceptable salts, hydrates, solvates, esters, N-oxides protected derivatives, individual isomers and mixture of isomers thereof or prodrugs thereof, in a quantity which is effective to promoting localized neochondrogenesis.
